# EUROPÄISCHE PATENTANMELDUNG

(11) **EP 2 924 601 A1**
(43) Veröffentlichungstag der Anmeldung: **30.09.2015**
(21) Anmeldenummer: 15000883.7
(22) Anmeldetag: 26.03.2015
(51) Int. Cl.: G06F 19/00

(54) **Vorrichtung zur Beatmung**

(30) Priorität: 28.03.2014 DE 102014004848
(71) Anmelder: Weinmann Geräte für Medizin GmbH + Co. KG, 22525 Hamburg (DE)
(72) Erfinder: Schwaibold, Matthias, 76228 Karlsruhe (DE)

(57) **Zusammenfassung**

Die Vorrichtung dient zur Beatmung und weist ein mit einer Steuerung verbundenes Gebläse auf. Sowohl die Steuerung als auch das Gebläse sind in einem Gehäuse angeordnet. Die Steuerung ist mit mindestens einer Anzeigeeinrichtung sowie mindestens einem Bedienelement verbunden.

## Beschreibung

Die Erfindung betrifft eine Vorrichtung zur Beatmung, die ein mit einer Steuerung verbundenes Gebläse aufweist, wobei sowohl die Steuerung als auch das Gebläse in einem Gehäuse angeordnet sind, und wobei die Steuerung mit mindestens einer Anzeigeeinrichtung sowie mindestens einem Bedienelement verbunden ist.

Derartige Vorrichtungen zur Beatmung werden typischerweise zur Energieversorgung an ein Versorgungsnetz angeschlossen und über einen Beatmungsschlauch mit einer Beatmungsmaske verbunden. Ein Patient legt die Beatmungsmaske während der Durchführung der Beatmung im Gesichtsbereich an.

Beispielsweise ist es bekannt, derartige Vorrichtungen zur Beatmung zur Durchführung einer CPAP-Beatmung oder einer APAP-Beatmung zu verwenden. Grundsätzlich können von der Steuerung des Gerätes beliebige Beatmungsabläufe vorgegeben werden.

Aufgabe der vorliegenden Erfindung ist es, eine Vorrichtung der einleitend genannten Art derart zu konstruieren, dass eine verbesserte Nutzungsqualität bereitgestellt wird.

Die erfindungsgemäße Vorrichtung zur Beatmung, weist ein mit einer Steuerung verbundenes Gebläse auf, wobei sowohl die Steuerung als auch das Gebläse in einem Gehäuse angeordnet sind, und wobei die Steuerung mit mindestens einer Anzeigeeinrichtung sowie mindestens einem Bedienelement verbunden ist, wobei die Anzeigeeinrichtung als ein Display ausgebildet ist, dass eine Touchscreen-Funktion aufweist und das unter Verwendung einer symbolbasierten Bedienstruktur derart als Eingabeeinrichtung verwendbar ist, dass jeweilige Symbole eine zugeordnete Funktion visualisieren und dass von der Steuerung eine hierarchisch strukturierte Bedienbarkeit generiert ist.

Die erfindungsgemäße Vorrichtung ist auch dadurch gekennzeichnet, dass über das Display unterschiedliche Sprachen auswählbar sind.

Die erfindungsgemäße Vorrichtung ist auch dadurch gekennzeichnet, dass eine Auswahlmöglichkeit von unterschiedlichen Sprachen für ein Patientenmenü und ein Expertenmenü vorgesehen sind, wobei über die Eingabetaste die Sprache für das betreffende Menü ausgewählt wird und die Eingabetaste schaltlogisch mit einem Sprachauswahlmenü verbunden ist, in dem der Anwender die Sprache auswählen kann, wobei die Eingabetaste zudem schaltlogisch mit dem Sprachspeicher verbunden ist und wobei nach Bestätigung der gewählten Sprache, diese aus dem Speicher geladen und angewendet wird.

Die erfindungsgemäße Vorrichtung ist auch dadurch gekennzeichnet, dass im Bereich des Displays mehrere Eingabetasten vorgesehen sind, die zum Empfangen einer Benutzereingabe konfiguriert sind, wobei das Display schaltlogisch mit den Tasten verbunden ist und mehrere zugeordnete Informationsfelder aufweist, wobei jedes Informationsfeld einer entsprechenden Eingabetaste zugeordnet ist und dazu konfiguriert ist, graphische Informationen anzuzeigen, die verschiedene Funktionen der entsprechenden Eingabetasten angeben, wobei das Informationsfeld eingerichtet ist graphisch anzugeben, ob ein einen Parameter oder eine Einstellung des Beatmungsgerätes betreffender, ausgewählter Wert mittels zumindest einer Eingabetaste verändert werden kann oder nicht.

Die erfindungsgemäße Vorrichtung ist auch dadurch gekennzeichnet, dass das Display mit einer Auswertungseinheit zur Bereitstellung mindestens einer qualitativen Therapieinformation verbunden ist.

Die erfindungsgemäße Vorrichtung ist auch dadurch gekennzeichnet, dass das Beatmungsgerät einen internen Datenspeicher für eine permanente Speicherung von Betriebsdaten aufweist.

Die erfindungsgemäße Vorrichtung ist auch dadurch gekennzeichnet, dass auswählbare Betriebsdaten auf einer Speicherkarte speicherbar sind.

Die erfindungsgemäße Vorrichtung ist auch dadurch gekennzeichnet, dass im Bereich des Displays mehrere Eingabetasten vorgesehen sind, die zum Empfangen einer Benutzereingabe konfiguriert sind wobei das Display schaltlogisch mit den Tasten verbunden ist und mehrere zugeordnete Informationsfelder aufweist, wobei jedes Informationsfeld einer entsprechenden Eingabetaste zugeordnet ist und dazu konfiguriert ist, graphische Informationen anzuzeigen, die verschiedene Funktionen der entsprechenden Eingabetasten angeben, wobei das Informationsfeld eingerichtet ist graphisch anzugeben, wie viele Tage mit Speicherdaten auf den externen Speicher (eine SD-Karte oder ein USB-Speicher) übertragen werden sollen.

Die erfindungsgemäße Vorrichtung ist auch dadurch gekennzeichnet, dass das Beatmungsgerät zur Durchführung einer Dichtigkeitsprüfung einer Beatmungsmaske ausgebildet ist und dass ein entsprechendes Prüfungsergebnis im Bereich des Displays visualisierbar ist.

Die erfindungsgemäße Vorrichtung ist auch dadurch gekennzeichnet, dass ein während der Durchführung der Prüfung auf Maskendichtigkeit verwendeter Druck über das Display vorgebbar ist.

Die erfindungsgemäße Vorrichtung ist auch dadurch gekennzeichnet, dass nach Betätigung der Eingabetaste, insbesondere die Durchführung eines Prüfvorganges für die Maskendichtigkeit während der Therapie im Expertenmenü erfolgt, wobei im Bereich des Displays mehrere Eingabetasten vorgesehen sind, die zum Empfangen einer Benutzereingabe konfiguriert sind und wobei das Display schaltlogisch mit den Tasten verbunden ist und mehrere zugeordnete Informationsfelder aufweist wobei jedes Informationsfeld einer entsprechenden Eingabetaste zugeordnet ist und dazu konfiguriert ist, graphische Informationen anzuzeigen, die verschiedene Funktionen der entsprechenden Eingabetasten angeben, wobei zumindest ein Informationsfeld mit zugeordneter Eingabetaste vorgesehen ist, wobei das Informationsfeld eingerichtet ist graphisch anzugeben, ob ein einen Parameter oder eine Einstellung des Beatmungsgerätes betreffender, ausgewählter Wert mittels zumindest einer Eingabetaste verändert werden kann oder nicht und eine Veränderung dieses Zustandes über eine Betätigung der Eingabetaste erreicht werden kann, die dem Informationsfeld zugeordnet ist, wobei nach einer entsprechenden Betätigung der Eingabetaste das Informationsfeld einen geschlossenen Zustand visualisiert und zumindest ausgewählte Werte die den Betrieb des Beatmungsgerätes betreffen, daraufhin nicht mehr veränderbar sind.

Die erfindungsgemäße Vorrichtung ist auch dadurch gekennzeichnet, dass über das Display mindestens eine Energiesparoption auswählbar und konfigurierbar ist.

Die erfindungsgemäße Vorrichtung ist auch dadurch gekennzeichnet, dass über das Display mindestens eine Information zur Gerätefunktion und/oder Gerätebedienung visualisierbar ist.

Das erfindungsgemäße Verfahren zur Anzeige von Benutzerschnittstelleninformationen für ein Beatmungsgerät, weist ein mit einer Steuerung verbundenes Gebläse auf, wobei sowohl die Steuerung als auch das Gebläse in einem Gehäuse angeordnet sind, und wobei die Steuerung mit mindestens einer Anzeigeeinrichtung sowie mindestens einem Bedienelement verbunden ist, dadurch gekennzeichnet, das die Anzeigeeinrichtung als ein Display ausgebildet ist, dass eine Touchscreen-Funktion aufweist und das unter Verwendung einer symbolbasierten Bedienstruktur derart als Eingabeeinrichtung verwendbar ist, dass jeweilige Symbole eine zugeordnete Funktion visualisieren und dass von der Steuerung eine hierarchisch strukturierte Bedienbarkeit generiert ist.

Das erfindungsgemäße Verfahren ist auch dadurch gekennzeichnet, dass im Bereich des Displays mehrere Eingabetasten vorgesehen sind, die zum Empfangen einer Benutzereingabe konfiguriert sind und wobei das Display schaltlogisch mit den Tasten verbunden ist und mehrere zugeordnete Informationsfelder aufweist wobei jedes Informationsfeld einer entsprechenden Eingabetaste zugeordnet ist und dazu konfiguriert ist, graphische Informationen anzuzeigen, die verschiedene Funktionen der entsprechenden Eingabetasten angeben, wobei zumindest ein Informationsfeld mit zugeordneter Eingabetaste vorgesehen ist, wobei das Informationsfeld eingerichtet ist graphisch anzugeben, ob ein einen Parameter oder eine Einstellung des Beatmungsgerätes betreffender, ausgewählter Wert mittels zumindest einer Eingabetaste verändert werden kann.

In den Zeichnungen sind Ausführungsbeispiele der Erfindung schematisch dargestellt. Es zeigen:
- Fig. 1: Eine perspektivische Darstellung eines Gerätes mit schräg angeordnetem Display,
- Fig. 2: eine perspektivische Darstellung einer geänderten Gerätekonfiguration,
- Fig. 3: eine perspektivische Darstellung einer Fronteinheit und einer montierten Gebläsebox,
- Fig. 3a: eine weitere perspektivische Darstellung einer Fronteinheit und einer montierten Gebläsebox,
- Fig. 4: eine perspektivische Darstellung eines innerhalb der Gebläsebox angeordneten Gebläses, wobei das Gebläse von einer umlaufenden Membranstruktur gehaltert ist,
- Fig. 4a: eine weitere perspektivische Darstellung des von einer umlaufenden Membran gehalterten Gebläses,
- Fig. 5: eine perspektivische Darstellung einer teilweise auseinandergenommenen Gebläsebox mit eingezeichneter Luftführung,
- Fig. 6: eine Darstellung entsprechend Fig. 5 ohne eingezeichnete Luftführung,
- Fig. 7: eine perspektivische Innenansicht eines Gehäuseteiles,
- Fig. 8: eine perspektivische Darstellung zur Veranschaulichung einer Halterung einer als Display ausgebildeten Anzeigeeinrichtung innerhalb des Gehäuses unter Verwendung eines Rahmens,
- Fig. 9: eine weitere Darstellung des Displays und des Halterahmens,
- Fig. 10: eine kombinierte Darstellung des Displays und des Halterahmens,
- Fig. 11: eine weitere perspektivische Darstellung des Beatmungsgerätes mit zugeordneter externer Bedieneinheit und
- Fig. 12: eine Draufsicht auf eine innerhalb des Gerätegehäuses angeordnete Steuerungsplatine mit Druckmessschlauch,
- Fig. 13: eine Draufsicht auf das Display in einem Ruhezustand des Beatmungsgerätes, in dem eine automatische Prüfung auf eine Dichtigkeit der angelegten Atemmaske erfolgt,
- Fig. 14: eine Darstellung des Displays in einem aktiven Grundzustand, während dessen eine Maskendichtigkeit geprüft wird,
- Fig. 15: eine Anzeige des Displays in einer Menüebene zur Auswahl zu verwendender Sprachen,
- Fig. 16: eine Darstellung des Displays in einer Menüebene zur Abspeicherung von Daten auf eine Speicherkarte,
- Fig. 17: eine Anzeige des Displays in einer Menüebene zur optionalen Auswahl eines Energiesparmodus,
- Fig. 18: eine Anzeige des Displays zur Visualisierung einer qualitativen Bewertung der durchgeführten Therapie,
- Fig. 19: mehrere Anzeigen des Displays für detaillierte Bewertungen der durchgeführten Therapie und
- Fig. 20: eine Anzeige des Displays zur Ermöglichung einer Auswahl von beispielhaften Demonstrationsdarstellungen zur Visualisierung von unterschiedlichen Funktionsmodi.

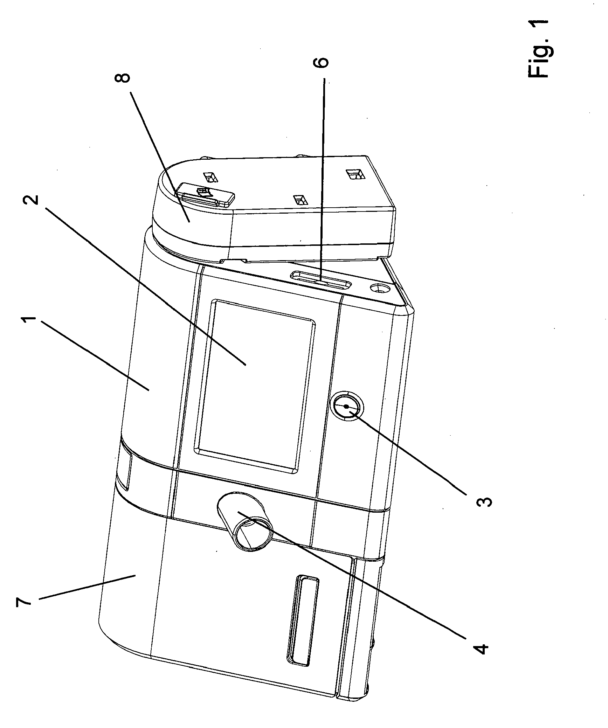

Fig. 1 zeigt ein Beatmungsgerät (1). Das Beatmungsgerät (1) ist mit einer Anzeigeeinrichtung (2), mindestens einem Bedienelement (3) sowie einem Schlauchanschluss (4) versehen.

Der Schlauchanschluss (4) dient typischerweise zur Verbindung mit einem nicht dargestellten Atemgasschlauch, der im Bereich seiner dem Schlauchanschluss (4) abgewandten Ausdehnung mit einer ebenfalls nicht dargestellten Atemmaske verbunden ist. Die nicht dargestellte Atemmaske dient zur Atemgasversorgung eines ebenfalls nicht dargestellten Patienten.

Das Beatmungsgerät (1) ist typischerweise mit mindestens einer Kommunikationsschnittstelle (6) versehen.

Bei dem dargestellten Ausführungsbeispiel ist das Beatmungsgerät (1) mit einem Atemgasbefeuchter (7) gekoppelt. Das Beatmungsgerät (1) kann optional sowohl mit als auch ohne den Atemgasbefeuchter (7) betrieben werden.

Gemäß der im Fig. 1 dargestellten Ausführungsform weist das Gehäuse des Beatmungsgerätes (1) in einer senkrechten Schnittebene eine dreieckartige Querschnittfläche auf.

Gemäß der Ausführungsform in Fig. 1 können an das Beatmungsgerät (1) bedarfsabhängig Zusatzelemente (8) angekoppelt werden, die mindestens eine weitere Gerätefunktion bereitstellen.

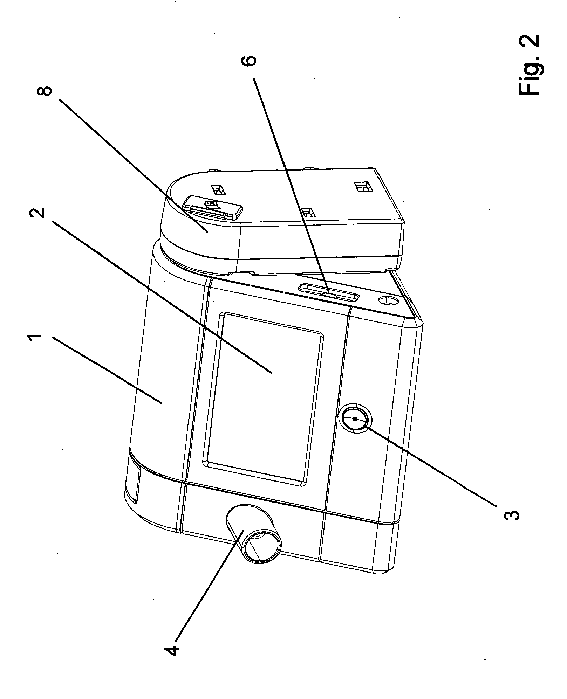

Gemäß der in Fig. 2 dargestellten Ausführungsform ist bei der Geräteanordnung gemäß Fig. 1 der Atemgasbefeuchter (7) vom Beatmungsgerät (1) abgenommen worden.

Bei einer Zusammenschau von Fig. 1 und Fig. 2 ist erkennbar, dass unabhängig von der Verwendung eines Atemgasbefeuchters (7) immer der gleiche Schlauchanschluss (4) verwendbar ist. Es ist somit nicht erforderlich, abhängig von der Gerätefunktion den Beatmungsschlauch umzustecken.

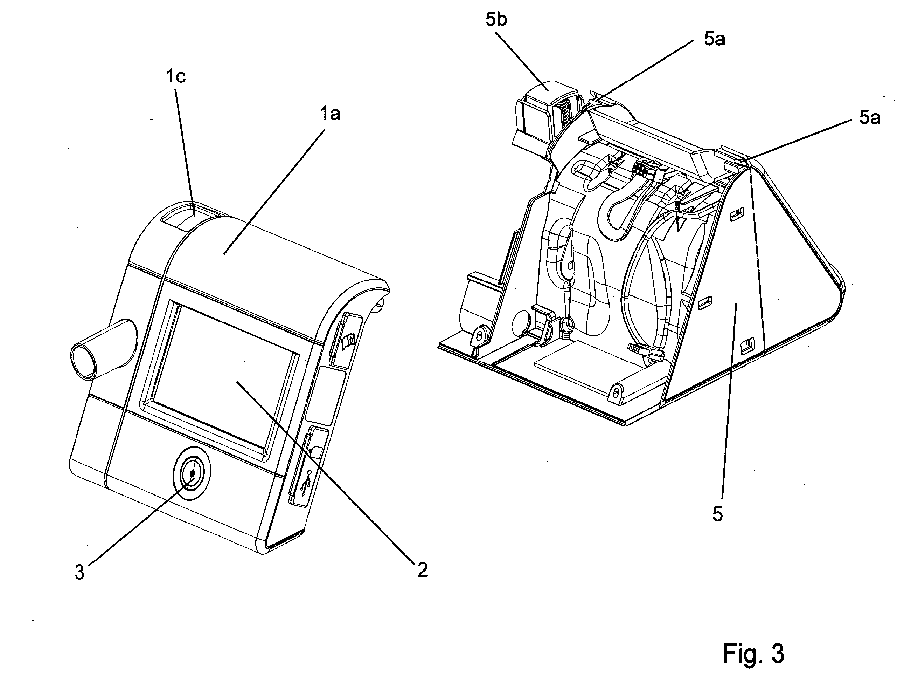

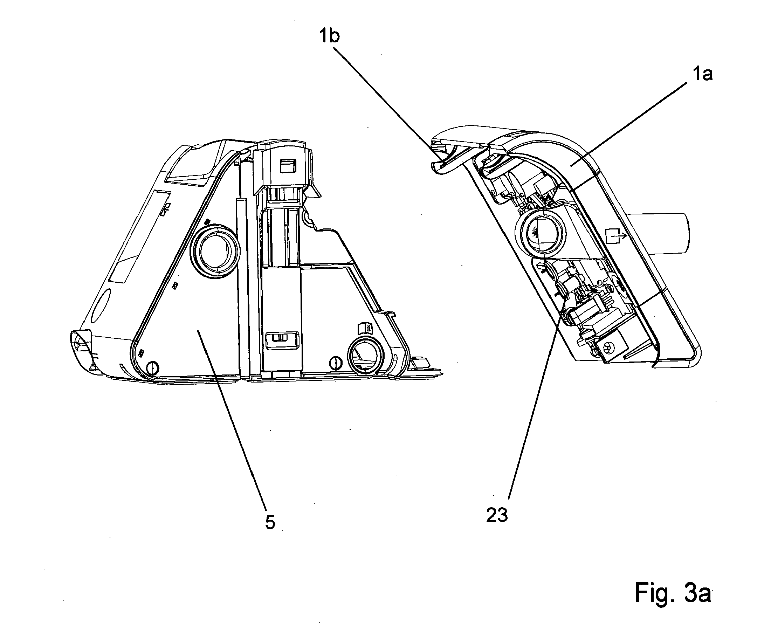

Fig. 3 veranschaulicht, dass das Beatmungsgerät (1) hinsichtlich seiner mechanischen Grundstruktur aus zwei Bauelementen besteht. Dieses sind eine Fronteinheit (1a) mit dem Bedienelement (3) sowie der Anzeigeeinrichtung (2) und der Gebläsebox (5), mit einem Vorderteil (16), einem Tragteil (11) mit dem Gebläse (9) und einem hinteren Teil (17). Das Vorderteil (16) und das Hinterteil (17) umschließen schalenartig einen Geräteinnenraum (18). In die Gehäuseteile (16) und (17) sind schalldämmende Materialeien (16', bzw. 17') bevorzugt aus einem Schaumstoff eingesetzt.

Fig. 3 und 3a zeigen das Beatmungsgerät (1) zerlegt in die Bauteile Gebläsebox (5) und Fronteinheit (1a). Aus den Figuren wird die einfache Handhabung bei der Montage der Bauteile ersichtlich. Die Fronteinheit (1a) enthält u.a. neben dem Gehäuse eine Anzeigeeinrichtung (2) (beispielsweise als Touchscreen (13)), ein Bedienelement (3) und eine Steuerplatine (23). Die Montage der Fronteinheit (1a) mit der Gebläsebox (5) erfolgt über Haken (1b) an der Froneinheit (1a), die hinter Bereiche (5a) der Gebläsebox (5) greifen. Die Fronteinheit wird über den Drehpunkt aus den Haken (1b) und den Bereichen (5a) eingeschwenkt. Zusätzlich greift ein an der Gebläsebox (5) angeordneter Schnapper (5b) in eine Öffnung (1c) der Fronteinheit (1a). Zur Sicherung wird die Fronteinheit (1a) lediglich mit 2 Schrauben mit der Gebläsebox (5) verbunden und bildet so das Gehäuse des Beatmungsgerätes (1). Zum Lösen der Fronteinheit (1a) von der Gebläsebox (5) müssen nur die Schrauben gelöst werden und der Schnapper (5b) entriegelt werden.

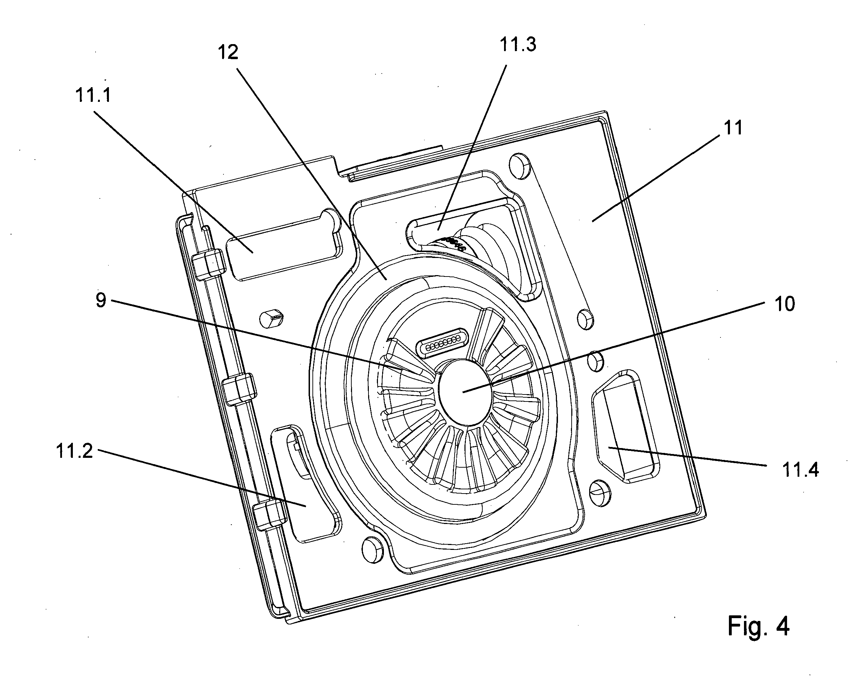

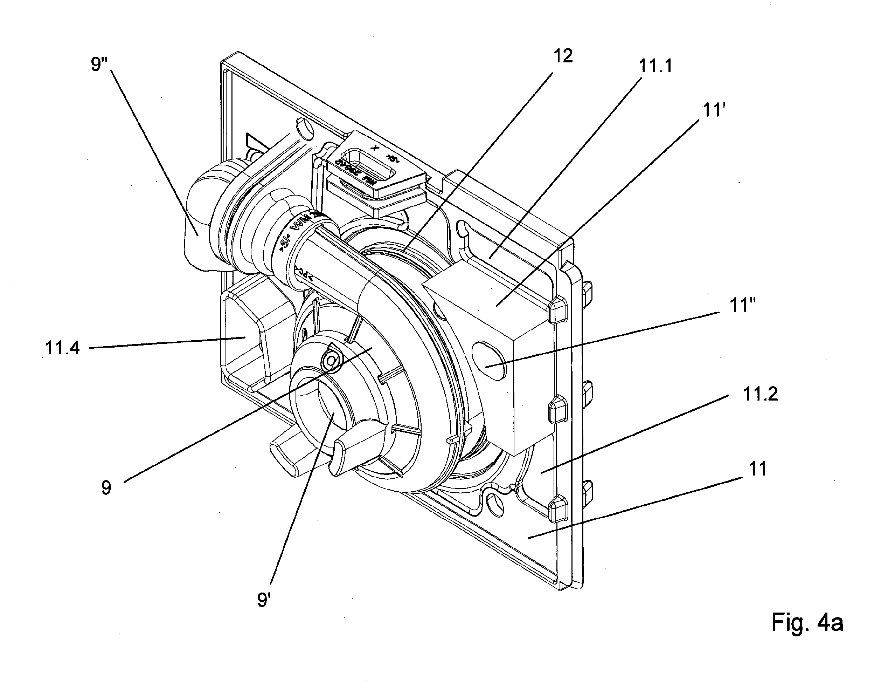

Gemäß der Ausführungsform in Fig. 4 ist innerhalb der Gebläsebox (5) ein Gebläse (9) angeordnet. Das Gebläse (9) wird typischerweise von einem Elektromotor (10) angetrieben.

Zur Unterstützung einer einfachen Montage und Demontage kann das Gebläse (9) von einem Tragteil (11) gehaltert sein, das in die Gebläsebox (5) einsetzbar ist.

Zur Unterstützung einer Körperschallentkopplung ist das Gebläse (9) durch ein elastisches Kopplungselement (12) mit dem Tragteil (11) verbunden. Bevorzugt sind Tragteil (11) und Kopplungselement (12) einteilig ausgeführt. Diese können beispielsweise aus einem elastomeren Material ausgeführt sein. Gemäß einer bevorzugten Ausführungsform umgibt das Kopplungselement (12) das Gebläse (9) rahmenartig. In Abhängigkeit von den jeweiligen Anwendungsanforderungen kann das Kopplungselement (12) ringartig gestaltet sein. Vorzugsweise weist das Kopplungselement (12) eine membranartige Gestaltung auf, beispielsweise ähnlich zu elastischen Aufhängungselementen, die bei Lautsprechern verwendet werden.

Das Tragteil (11) mit dem Kopplungselement (12) besteht bevorzugt aus einem elastischen Material, beispielsweise aus Silikon.

Das Tragteil (11) enthält außer dem Kopplungselement (12) verschiedene Luftöffnungen um einen längeren Luftweg und somit eine weitere Schallreduzierung zu erzielen.

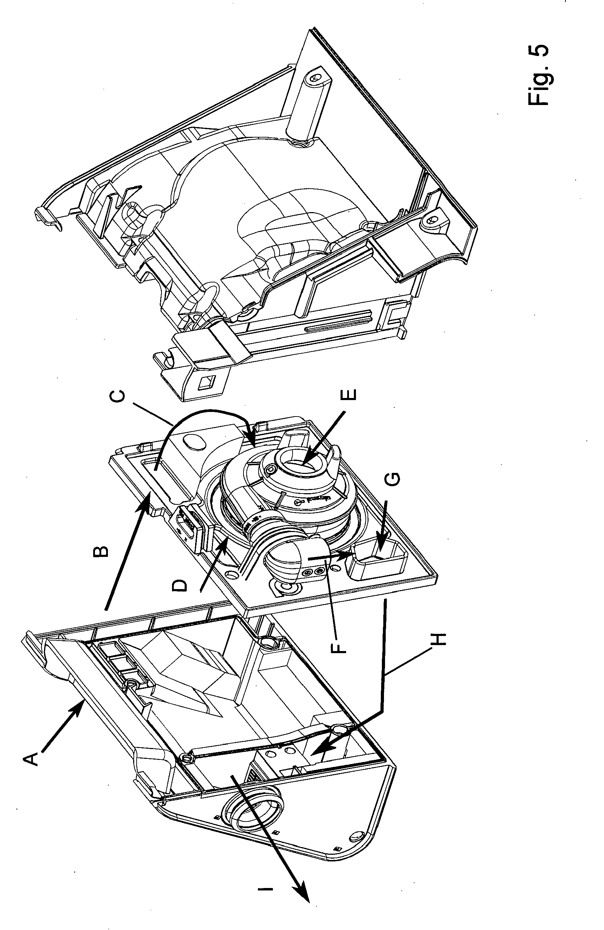

Das Tragteil (11) dient neben der Aufnahme des Gebläses (9) und der gezielten Gasströmung durch den Geräteinnenraum (18) der Gebläsebox (5) welches in Fig. 5 veranschaulicht wird auch der Abdichtung zwischen den Gehäuseteilen (16) und (17). Die Außenkontur des Tragteiles (11) nimmt die Außenkontur der Gehäuseteile (16 und 17) auf und dichtet so im Randbereich zwischen den Gehäuseteilen (16 und 17) ab.

Fig. 4a zeigt das Tragteil (11) in einer weiteren Ansicht. Hier wird der Ansaugbereich (9') und der Luftauslass (9") des Gebläses (9) sichtbar. Ein zusätzliches Schaumstoffelement (11'), welches mittels eines Zapfens (11") auf dem Tragteil (11) gehalten wird, dient der Luftumlenkung und somit der Schallreduzierung.

Fig. 5 veranschaulicht eine typische Luftströmung durch die Gebläsebox (5) hindurch. Gemäß A wird Umluft durch eine Ansaugöffnung im Hinterteil (16) angesaugt und strömt gemäß B durch die Öffnung (11.1) im Tragteil (11). Gemäß C wird die Luftströmung durch eine Kammer (17.1) im Vorderteil (17) vertikal umgelenkt und tritt durch die Öffnung (11.2) im Tragteil (11) zurück in das Hinterteil (16) in die Luftkammer im Bereich (16.1). Dort wird die Luft diagonal umgelenkt und strömt gemäß D durch die Öffnung (11.3) und diagonal umgelenkt weiter gemäß E im Bereich (9') in das Gebläse (9) ein. Die vom Gebläse (9) geförderte Luft verlässt das Gebläse durch den Luftauslass (9") gemäß F im Bereich (17.2) im Vorderteil (17) weiter gemäß G durch die Öffnung (11.4) im Tragteil (11). Gemäß H erreicht die Luftströmung das Hinterteil (16) im Bereich (16.2) durchströmt dort eine Flow-Messeinrichtung und wird gemäß I erneut umgelenkt und verlässt das Hinterteil (16) zu einem Schalldämpfer oder zum Atemgasbefeuchter.

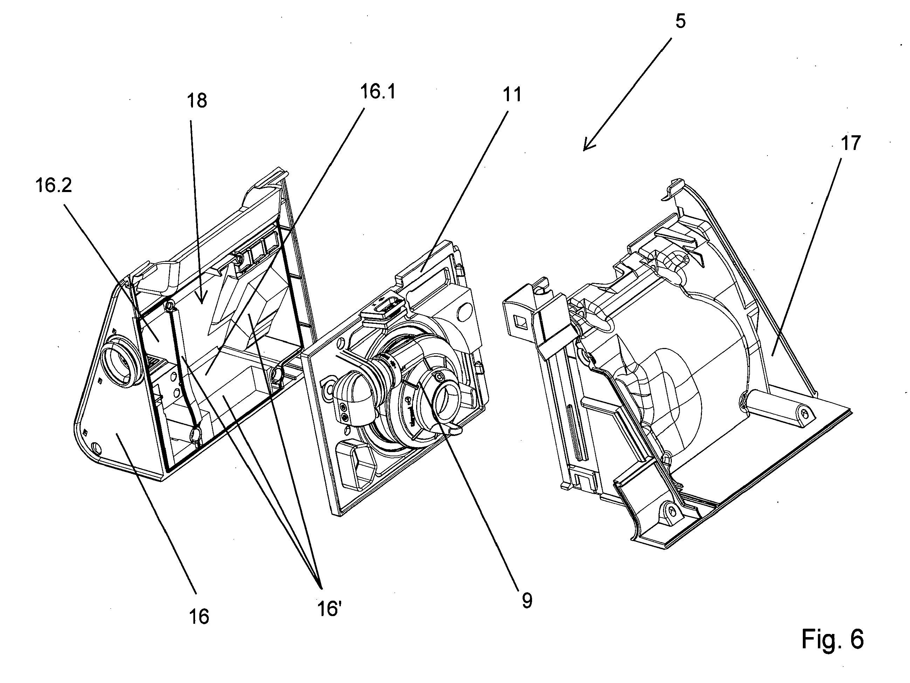

Fig. 6 zeigt die Bauteile gemäß Fig. 5 zur näheren Erläuterung der mechanischen Konstruktion ohne die eingezeichneten Strömungspfeile der Gasströmung.

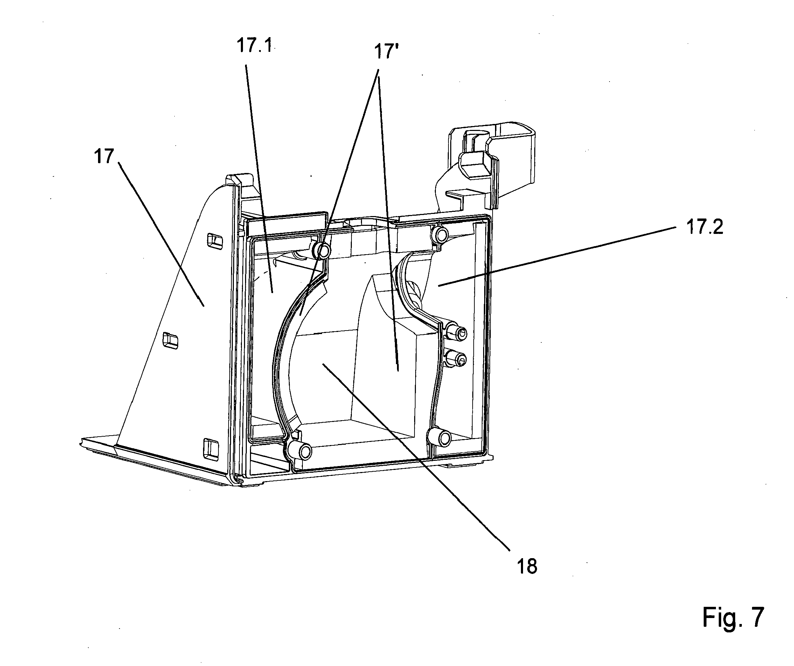

Fig. 7 zeigt den Gehäuseinnenraum (18) in dem Vorderteil (17) mit eingesetzten schalldämmenden Materialien (17') und den Luftkammern (17.1) und (17.2).

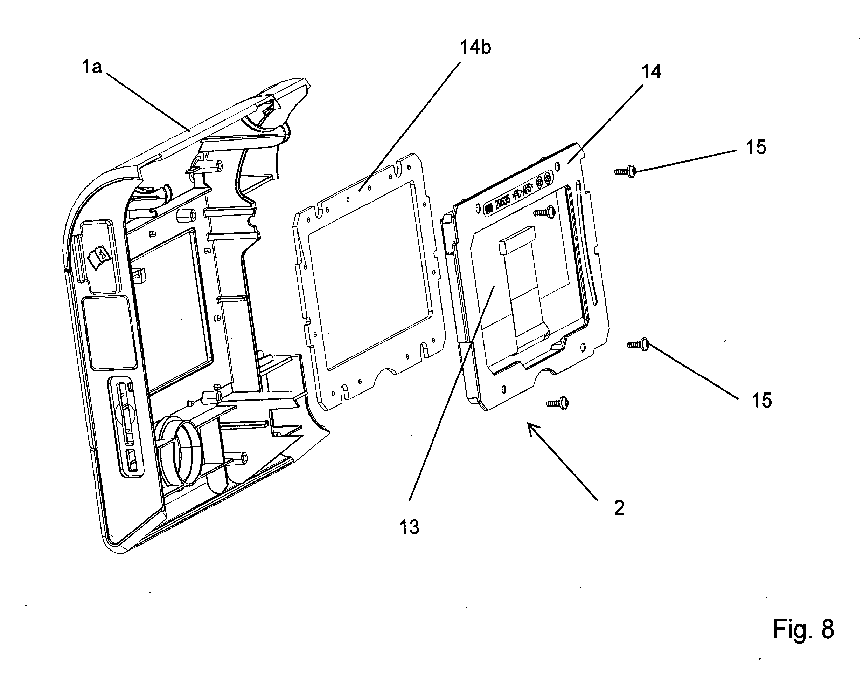

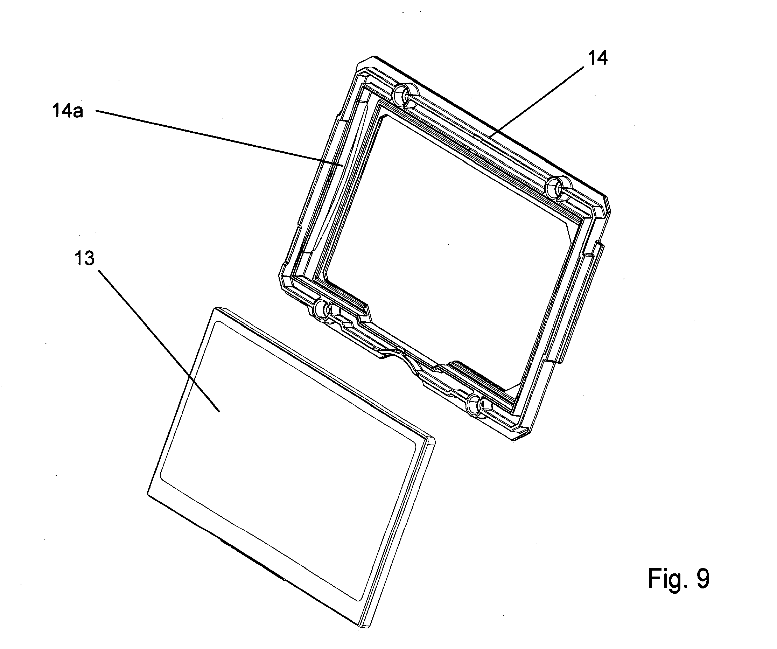

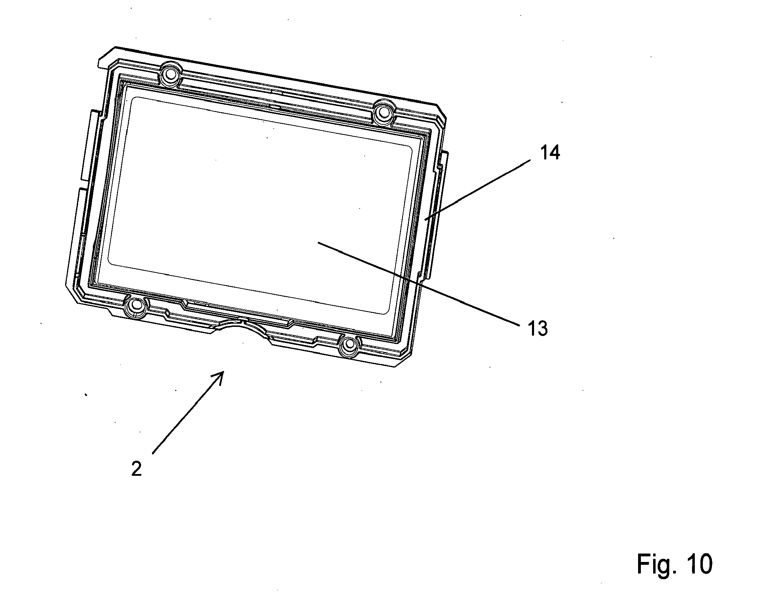

Gemäß der Ausführungsform in Fig. 8 ist die Anzeigeeinrichtung (2) als ein Display (13) ausgebildet. Das Display (13) ist plattenartig gestaltet und wird mittels eines rahmenartigen Halterungselements (14) innerhalb des Gehäuses (5) fixiert. Vorzugsweise ist daran gedacht, dass das Display (13) in das Halterungselement (14) eingesetzt und mittels eines Klemmbügels (14a) in dem Halterungselement (14) fixiert wird. Dieses dient der besseren Fixierung des Displays im Gehäuse, da das Display über Kopf in das Gehäuse montiert wird und ohne Klemmbügel verrutschen kann. Das Halterungselement (14) ist zudem so ausgelegt, dass es Fertigungstoleranzen des Displays (13) durch eine unterschiedliche Spannung des Klemmbügels (14a) ausgleichen kann. Die Einzelteile sind in Fig.9 und die vormontierte Einheit aus Display (13) und Halterungselement (14) ist in Fig. 10 dargestellt. Die vormontierte Einheit wird unter Verwendung von Schrauben (15) gegenüber dem Gehäuse (5) verspannt. Vorzugsweise ist zwischen dem Display (13) und dem Gehäuse (1a) eine Dichtung (14b) angeordnet. Bei Verwendung eines anderen Displays (13) z.B. eines anderen Herstellers braucht in dieser Ausführungsform nur das Halterungselement (14) an das Display angepasst werden, die Gehäusekonstruktion kann beibehalten werden.

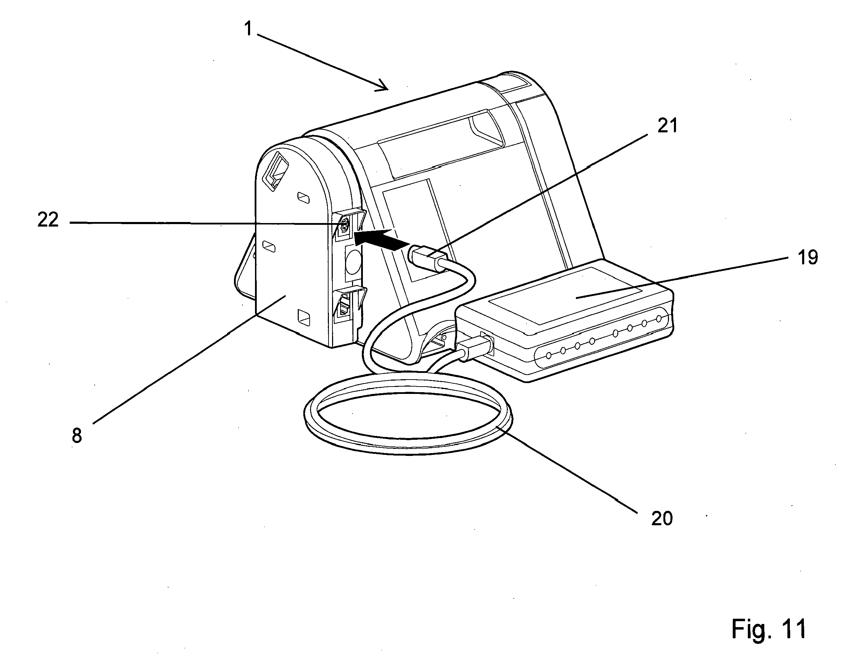

Fig. 11 zeigt eine Ausführungsform, bei der das Beatmungsgerät (1) mit einem als Kommunikationsmodul ausgebildeten Zusatzelement (8) versehen ist. Das Zusatzelement (8) dient hier zur Verbindung mit einem Polysomnographie-Gerät (PSG) (19). Eine Ankopplung des PSG (19) kann unter Verwendung eines Kabels (20) erfolgen, das über einen Stecker (21) mit einer Schnittstelle (22) des Zusatzelementes (8) koppelbar ist.

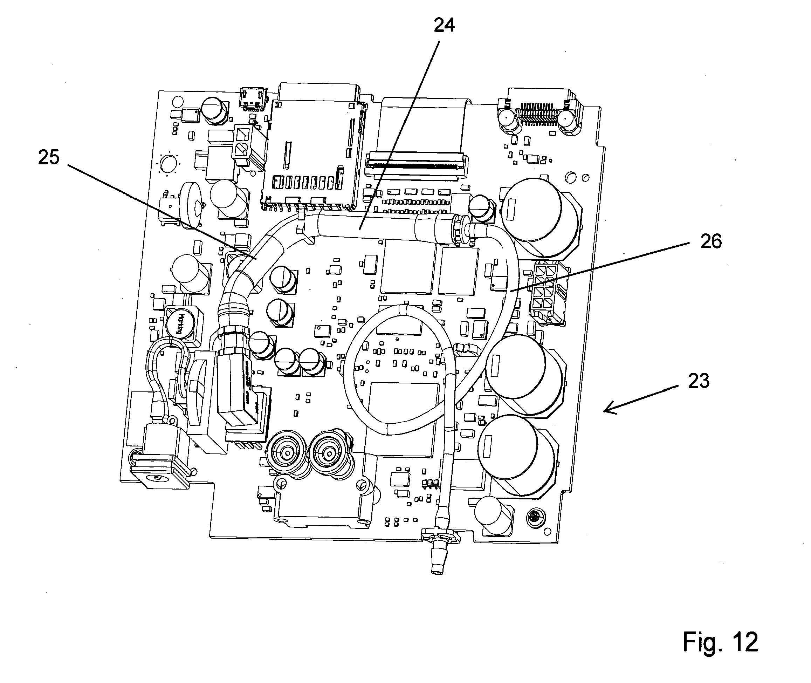

Fig. 12 zeigt eine Draufsicht auf eine Steuerungsplatine (23), die innerhalb der Fronteinheit (1a) des Beatmungsgerätes (1) anordbar ist. Die Steuerungsplatine (23) unterstützt eine Druckmessung und ist zur Weiterleitung des Druckes mit einem steckbaren Schlauch (24) versehen.

Gemäß dem dargestellten Ausführungsbeispiel besteht der Schlauch (24) aus einem ersten Schlauchabschnitt (25) mit einem ersten Durchmesser aus und einem zweiten Schlauchabschnitt (26)mit einem zweiten Durchmesser. Der erste Durchmesser ist größer als der zweite Durchmesser.

Durch die Kombination des ersten Schlauchabschnittes (25) und des zweiten Schlauchabschnittes (26) wird eine Signalfilterung bereitgestellt, die insbesondere im Drucksignal vorhandene Oberwellen herausfiltert. Alternativ zur mechanischen Realisierung des Filters über die Schlauchabschnitte (25, 26) ist grundsätzlich auch eine elektrische, elektronische oder Softwarebasierte Filterung möglich.
In Kombination mit mindestens einem der vorstehend erläuterten Bauelemente und/oder Verfahrensschritte ist insbesondere daran gedacht, die Kommunikationsschnittstelle (6) als einen USB-Anschluss oder Mikro-USB-Anschluss auszubilden. Darüber hinaus ist insbesondere auch daran gedacht, die Kommunikationsschnittstelle (6) mit einem Detektor auszustatten oder zu verbinden, der erkennt, ob an die Kommunikationsschnittstelle (6) ein Kabel und/oder ein Stecker angeschlossen ist.

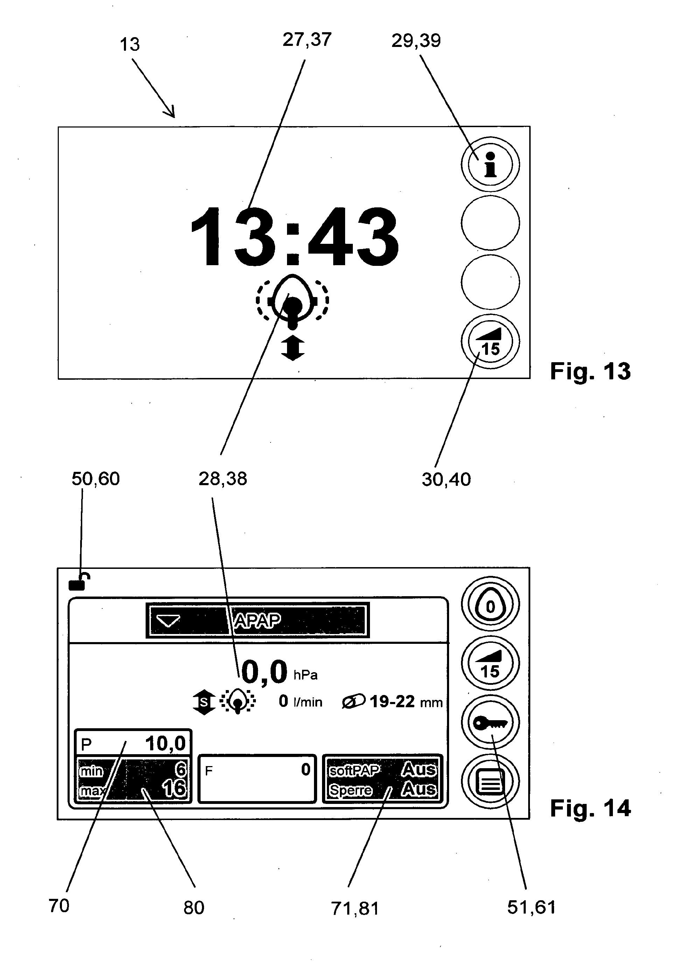

Fig. 13 veranschaulicht eine mögliche Anzeige des Displays (13) in einem Grundzustand des Beatmungsgerätes (1). Da das Beatmungsgerät häufig während der Nacht auf einem Nachttisch des Patienten aufgestellt ist, zeigt das Display (13) im dargestellten Ausführungsbeispiel die aktuelle Uhrzeit (27, 37) an. Zusätzlich erfolgt über eine symbolhafte Visualisierung eine Anzeige über eine durchgeführte Maskendichtigkeit (28, 38) während der Therapie im Patientenmenü. Das entsprechende Funktionssymbol ist unterhalb der angezeigten Uhrzeit zu erkennen.
Das Display (13) ist hier als Benutzerschnittstelle, beispielsweise als Touchscreen, des Beatmungsgerätes (1) ausgeführt. Im Bereich des Displays (13) sind mehrere Eingabetasten (27, 28, 29, 30) vorgesehen, die zum Empfangen einer Benutzereingabe konfiguriert sind. Das Display (13) ist schaltlogisch mit den Tasten (27 - 30) verbunden und weist mehrere Informationsfelder (37, 38, 39, 40) auf wobei jedes Informationsfeld einer entsprechenden Eingabetaste (27, 28, 29, 30) zugeordnet ist und dazu konfiguriert ist, graphische Informationen anzuzeigen, die verschiedene Funktionen der entsprechenden Eingabetasten (27, 28, 29, 30) angeben. Die Eingabetasten (27, 28, 29, 30) sind berührempfindliche Flächen im Bereich des Displays (13)
Erfindungsgemäß ist lediglich eine weiteres Bedienelement (3) (siehe Figuren 1, 2, 3)vorgesehen. Dieses Bedienelement (3) ist mechanisch bedienbar und unterscheidet sich somit von den Berührflächen (27, 28, 29, 30) auf dem Display. Erfindungsgemäß wird das Beatmungsgerät über das Bedienelement(3) ein- und ausgeschaltet, sodass eine grundlegende, patientengerechte Therapie mittels lediglich einer Taste aktiviert werden kann. Dazu ist das Bedienelement (3) schaltlogisch mit dem Gebläsemotor verbunden und aktiviert diesen, sowie den Speicher um hinterlegte Therapiedaten, wie Druckwerte, abzurufen und anzuwenden. Ergänzend kann über die Eingabetasten (27, 28, 29, 30) eine Anpassung einzelner Werte, wie Druckwerte, erfolgen. Eine Bedienung über die Eingabetasten (27, 28, 29, 30) des Touchscreens (13) ist erfindungsgemäß jedoch nicht notwendig, um die Therapie zu starten.

Fig. 14 zeigt eine Anzeige des Displays (13) in einem aktiven Grundzustand des Beatmungsgerätes, nachdem der Eingangsbildschirm gemäß Fig. 13 verlassen wurde. Die Darstellung zeigt, nach Betätigung der Eingabetaste (28), insbesondere die Durchführung eines Prüfvorganges für die Maskendichtigkeit während der Therapie im Expertenmenü mit einer Angabe von l/min und in einem Zustand zum Verstellen des Druckes (80), um die Maskendichtigkeit auch bei höheren Druckwerten zu testen. Dies entspricht der kachelartigen Symboldarstellung in der Displayanzeige links unten mit P min/max. Im Bereich des Displays (13) sind wieder mehrere Eingabetasten (27 ...50, 51) vorgesehen, die zum Empfangen einer Benutzereingabe konfiguriert sind. Das Display (13) ist schaltlogisch mit den Tasten (27 - 51) verbunden und weist mehrere zugeordnete Informationsfelder (37 ... 61) auf wobei jedes Informationsfeld einer entsprechenden Eingabetaste zugeordnet ist und dazu konfiguriert ist, graphische Informationen anzuzeigen, die verschiedene Funktionen der entsprechenden Eingabetasten angeben. Die Eingabetasten sind berührempfindliche Flächen im Bereich des Displays (13). Erfindungsgemäß ist zumindest ein Informationsfeld (60, 61) mit zugeordneter Eingabetaste (50, 51) vorgesehen. Das Informationsfeld (50, 51) ist eingerichtet graphisch anzugeben, ob ein einen Parameter oder eine Einstellung des Beatmungsgerätes betreffender, ausgewählter Wert (80, 81) mittels zumindest einer Eingabetaste (70, 71) verändert werden kann oder nicht. Das Informationsfeld (60) veranschaulicht hier, dass die Veränderung der Werte möglich ist (geöffnetes Schloss). Eine Veränderung dieses Zustandes kann über eine Bestätigung der Eingabetaste (61) erreicht werden, die dem Informationsfeld (61) zugeordnet ist. Nach einer entsprechenden Betätigung der Eingabetaste (61) visualisiert das Informationsfeld (60) ein geschlossenes Schloss und es sind zumindest ausgewählte Werte (80, 81), die den Betrieb des Beatmungsgerätes betreffen, nicht mehr veränderbar.

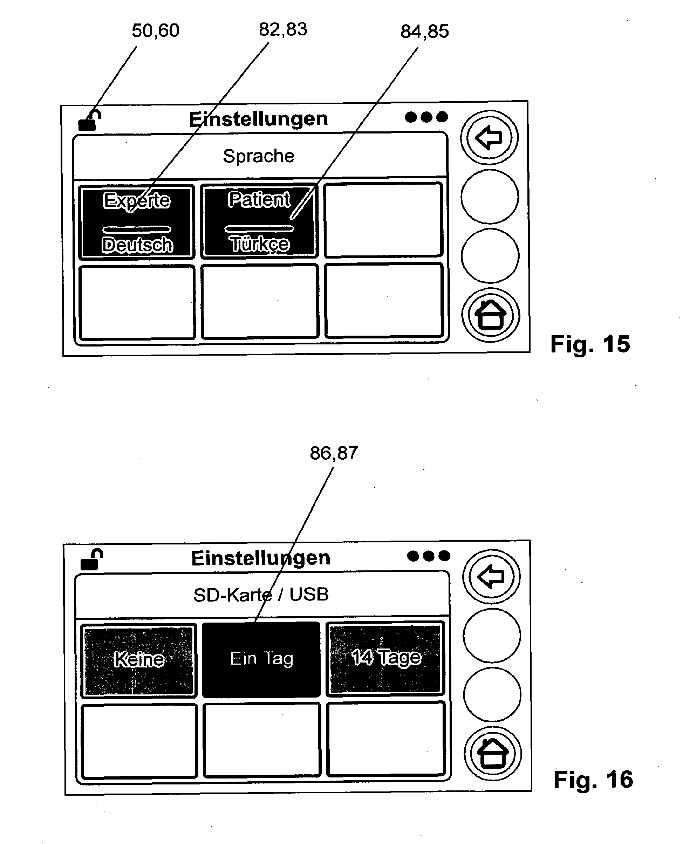

Fig. 15 zeigt eine Anzeige des Displays (13) zur Unterstützung einer Eingabe für die Auswahl einer im Patientenmenü und/oder Expertenmenü verwendeten Sprache. Im Bereich des Displays (13) sind wieder mehrere Eingabetasten (27 ...50 ...82, 84) vorgesehen, die zum Empfangen einer Benutzereingabe konfiguriert sind. Das Display (13) ist schaltlogisch mit den Tasten (27 - 84) verbunden und weist mehrere zugeordnete Informationsfelder (37 ... 85) auf wobei jedes Informationsfeld einer entsprechenden Eingabetaste zugeordnet ist und dazu konfiguriert ist, graphische Informationen anzuzeigen, die verschiedene Funktionen der entsprechenden Eingabetasten angeben. Die Eingabetasten sind berührempfindliche Flächen im Bereich des Displays (13). Erfindungsgemäß ist zumindest ein Informationsfeld mit zugeordneter Eingabetaste vorgesehen. Das Informationsfeld (50) ist eingerichtet graphisch anzugeben, ob ein einen Parameter oder eine Einstellung des Beatmungsgerätes betreffender, ausgewählter Wert (82, 84) mittels zumindest einer Eingabetaste (83, 85) verändert werden kann oder nicht. Das Informationsfeld (50) veranschaulicht hier, dass die Veränderung der Werte möglich ist (geöffnetes Schloss); was symbolisiert, dass das Gerät im Expertenmodus ist. Der Expertenmodus ist dadurch gekennzeichnet, dass hier viele grundlegende Einstellungen vorgenommen werden können, während im Patientenmodus lediglich beispielsweise Comfortfunktionen verändert werden können. Im Expertenmenü kann die Sprache eingestellt werden.
Die Auswahlmöglichkeit von unterschiedlichen Sprachen für das Patientenmenü und das Expertenmenü ermöglicht eine sichere Bedienung für den Experten und den Patienten. Die unterschiedlichen Sprachführungen in den Menüs sind unabhängig voneinander. Erfindungsgemäß wird damit dem Umstand Rechnung getragen, dass Arzt und Patient unterschiedliche Muttersprachen sprechen. Im dargestellten Ausführungsbeispiel ist deutsch für das Expertenmenü und türkisch als Sprache für das Patientenmenü auswählt. Dazu wird über die Eingabetaste (84) die Sprache für das Patientenmenü ausgewählt beziehungsweise dargestellt. Die Eingabetaste ist schaltlogisch mit einem Sprachauswahlmenü verbunden, in dem der Anwender die Sprache auswählen kann. Zudem ist die Eingabetaste (84) schaltlogisch mit dem Sprachspeicher verbunden. Nach Bestätigung der gewählten Sprache wird diese aus dem Speicher geladen und angewendet. In gleicher Weise erfolgt die Auswahl und Einstellung der Sprache für den Expertenmodus über die Taste (82). Eine derartige Menüstruktur unterstützt eine sichere Bedienung des Beatmungsgerätes (1) auch von Personen, die beispielsweise die deutsche Sprache nur teilweise beherrschen. Auch ein fachlicher Experte kann auf die veränderbaren Parametereinstellungen in seiner ausgewählten Muttersprache zugreifen.

Gemäß der Darstellung des Displays (13) in Fig. 16 wird von der Bedienstruktur die Möglichkeit geboten, die Therapiedaten auf einer Speicherkarte abzuspeichern. Dies kann beispielsweise eine SD-Karte oder ein USB-Speicher sein. Die entsprechende Speichermöglichkeit stellt sicher, dass keine Therapiedaten verlorengehen können. Die betreffenden Daten werden zusätzlich immer im Gerät gespeichert. Eine Kopie der im Gerät gespeicherten Daten wird bei einer entsprechenden Aktivierung durch das Menü auf die Speicherkarte geschrieben. Der Umfang der auf die Speicherkarte zu übertragenden Daten ist einstellbar. Im dargestellten Ausführungsbeispiel können beispielsweise die Daten für einen Tag oder für 14 Tage abgespeichert werden. Im Bereich des Displays (13) sind wieder mehrere Eingabetasten (27 ... 86) vorgesehen, die zum Empfangen einer Benutzereingabe konfiguriert sind. Das Display (13) ist schaltlogisch mit den Tasten (27 - 86) verbunden und weist mehrere zugeordnete Informationsfelder (37 ... 87) auf wobei jedes Informationsfeld einer entsprechenden Eingabetaste zugeordnet ist und dazu konfiguriert ist, graphische Informationen anzuzeigen, die verschiedene Funktionen der entsprechenden Eingabetasten angeben. Die Eingabetasten sind berührempfindliche Flächen im Bereich des Displays (13). Erfindungsgemäß ist zumindest ein Informationsfeld mit zugeordneter Eingabetaste vorgesehen. Das Informationsfeld (87) ist eingerichtet graphisch anzugeben, wie viele Tage mit Speicherdaten auf den externen Speicher (eine SD-Karte oder ein USB-Speicher) übertragen werden sollen. Die Eingabetaste (86) ist schaltlogisch mit dem Speicher für Therapiedaten verbunden. Eine Auswahl der Taste (86) veranlasst die Daten eines Tages (des letzten Tages) aus dem Speicher für Therapiedaten auf den externen Speicher zu kopieren.

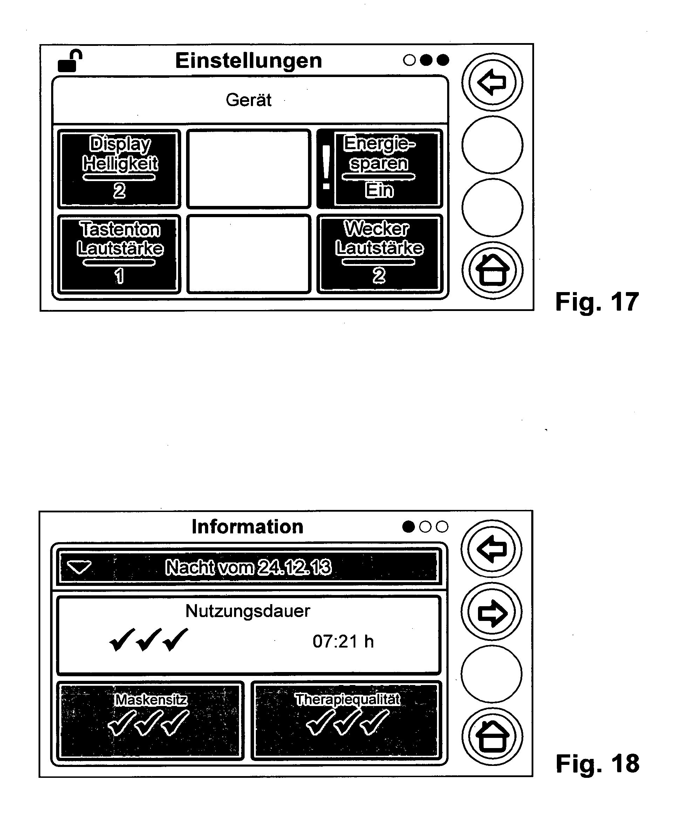

Gemäß der in Fig. 17 dargestellten Anzeige des Displays (13) ist es möglich, dass das Beatmungsgerät (1) nach einer vorgebbaren Zeit in einen Energiesparmodus überführt wird. Über die dargestellte Visualisierung auf dem als Touchscreen ausgebildeten Display (13) kann ein entsprechender Funktionsumfang innerhalb des Energiesparmodus ausgewählt werden. Zudem kann die Helligkeit des Displays in Stufen einstellbar sein. Auch kann die Lautstärke für einen Weckton in Stufen einstellbar sein.

Fig. 18 visualisiert eine Anzeige des Displays (13), um einem Patienten eine Information über die Qualität der durchgeführten Therapie zu übermitteln. Dazu bereitet das Beatmungsgerät die Daten für einen ausgewählten Zeitraum (hier eine Nacht) statistisch auf und gibt die Nutzungsdauer an. Zudem wird die Nutzungsdauer qualitativ bewertet und es wird eine symbolhafte graphische Ausgabe generiert, und räumlich benachbart zu der Nutzungsdauer angezeigt, die eine qualitative Aussage über die Nutzungsdauer wiedergibt oder veranschaulicht. Dazu sind Regeln im Gerät hinterlegt. Eine Nutzungsdauer von weniger als 3 Stunden pro Nacht wird so beispielsweise als nicht ausreichend bewertet, wobei eine symbolhafte graphische Ausgabe generiert, und räumlich benachbart zu der Nutzungsdauer angezeigt wird, die eine qualitative Aussage "nicht ausreichend" über die Nutzungsdauer wiedergibt oder veranschaulicht. Ebenso wird die Therapiequalität qualitativ bewertet und es wird eine symbolhafte graphische Ausgabe generiert und angezeigt die eine qualitative Aussage über die Therapiequalität wiedergibt oder veranschaulicht. Dazu sind Regeln im Gerät hinterlegt. Ein AHI von weniger als 1 pro Stunde wird so beispielsweise als ausreichend bewertet, wobei eine symbolhafte graphische Ausgabe generiert und angezeigt wird, die eine qualitative Aussage "ausreichend" über die Nutzungsdauer wiedergibt oder veranschaulicht. Bevorzugt fließen in die Bewertung der Therapiequalität mehrere Meßdaten ein.

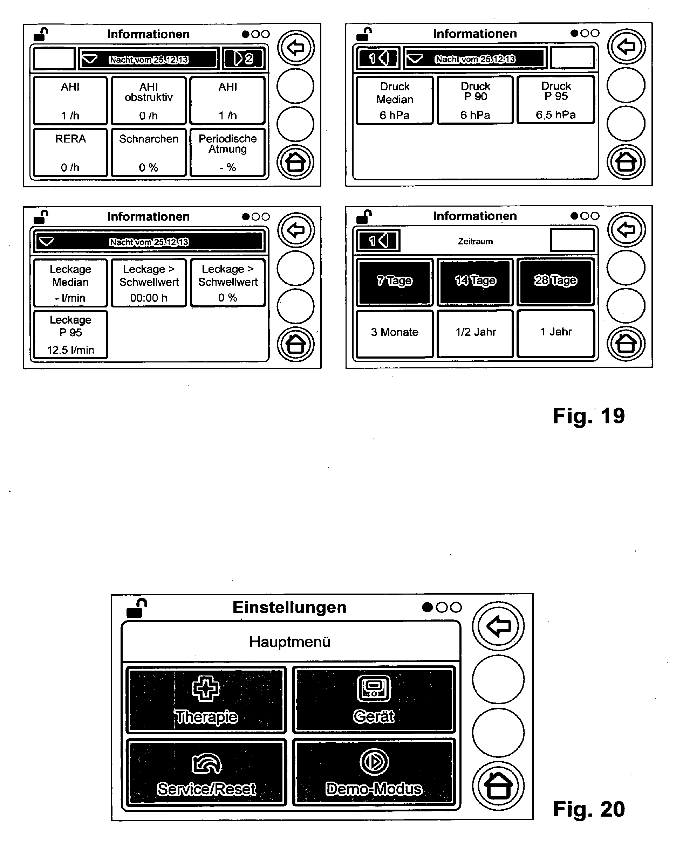

Fig. 19 veranschaulicht unterschiedliche Anzeigen des Displays (13), um einem Patienten weitere qualitative Informationen über die durchgeführte Therapie zu übermitteln. Beispielsweise fließen in die Bewertung der Therapiequalität gemäß Figur 18 mehrere Meßdaten ein wie Schnarchen und/oder Anteil periodischer Atmung und/oder mittlerer Therapie Druck und/oder Leckage. Dier Ermittlung und Speicherung der entsprechenden Daten erfolgt online im Gerät. Beispielsweise ist es möglich, die Therapiequalität für bestimmte Zeiträume bis zu einem Jahr zu visualisieren. Eine Beurteilung der Therapie ist so auch ohne spezielle Therapiesoftware möglich.

Gemäß der Anzeige des Displays (13) in Fig. 20 ist es möglich, Demonstrationsvisualisierungen über das Tastenfeld Demo-Modus zu aktivieren, mit denen einem Benutzer die Funktionalitäten erläutert werden. Beispielsweise ist es möglich, eine Demonstration für die Durchführung von Messvorgängen zu aktivieren. Ebenfalls ist es möglich, zu Demonstrationszwecken unterschiedliche Menüebenen oder Funktionsdarstellungen auszuwählen.

## Patentansprüche

1. Vorrichtung zur Beatmung, die ein mit einer Steuerung verbundenes Gebläse aufweist, wobei sowohl die Steuerung als auch das Gebläse in einem Gehäuse angeordnet sind, und wobei die Steuerung mit mindestens einer Anzeigeeinrichtung sowie mindestens einem Bedienelement verbunden ist, **dadurch gekennzeichnet**, das die Anzeigeeinrichtung (2) als ein Display (13) ausgebildet ist, dass eine Touchscreen-Funktion aufweist und das unter Verwendung einer symbolbasierten Bedienstruktur derart als Eingabeeinrichtung (2) verwendbar ist, dass jeweilige Symbole eine zugeordnete Funktion visualisieren und dass von der Steuerung eine hierarchisch strukturierte Bedienbarkeit generiert ist.

2. Vorrichtung nach Anspruch 1, **dadurch gekennzeichnet, dass**, das über das Display (13) unterschiedliche Sprachen auswählbar sind.

3. Vorrichtung nach Anspruch 1 und/oder 2, **dadurch gekennzeichnet, dass**, eine Auswahlmöglichkeit von unterschiedlichen Sprachen für ein Patientenmenü und ein Expertenmenü vorgesehen sind, wobei über die Eingabetaste (82, 84) die Sprache für das betreffende Menü ausgewählt wird und die Eingabetaste schaltlogisch mit einem Sprachauswahlmenü verbunden ist, in dem der Anwender die Sprache auswählen kann, wobei die Eingabetaste (82, 84) zudem schaltlogisch mit dem Sprachspeicher verbunden ist und wobei nach Bestätigung der gewählten Sprache, diese aus dem Speicher geladen und angewendet wird.

4. Vorrichtung nach Anspruch 2 und/oder 3, **dadurch gekennzeichnet, dass**, im Bereich des Displays (13) mehrere Eingabetasten (27 ...50 ...82, 84) vorgesehen sind, die zum Empfangen einer Benutzereingabe konfiguriert sind, wobei das Display (13) schaltlogisch mit den Tasten (27 - 84) verbunden ist und mehrere zugeordnete Informationsfelder (37 ... 85) aufweist, wobei jedes Informationsfeld einer entsprechenden Eingabetaste zugeordnet ist und dazu konfiguriert ist, graphische Informationen anzuzeigen, die verschiedene Funktionen der entsprechenden Eingabetasten angeben, wobei das Informationsfeld (50) eingerichtet ist graphisch anzugeben, ob ein einen Parameter oder eine Einstellung des Beatmungsgerätes betreffender, ausgewählter Wert (82, 84) mittels zumindest einer Eingabetaste (83, 85) verändert werden kann oder nicht.

5. Vorrichtung nach zumindest einem der vorhergehenden Ansprüche, **dadurch gekennzeichnet, dass** das Display (13) mit einer Auswertungseinheit zur Bereitstellung mindestens einer qualitativen Therapieinformation verbunden ist.

6. Vorrichtung nach zumindest einem der vorhergehenden Ansprüche, **dadurch gekennzeichnet, dass** das Beatmungsgerät (1) einen internen Datenspeicher für eine permanente Speicherung von Betriebsdaten aufweist.

7. Vorrichtung nach zumindest einem der vorhergehenden Ansprüche, **dadurch gekennzeichnet, dass** auswählbare Betriebsdaten auf einer Speicherkarte speicherbar sind.

8. Vorrichtung nach Anspruch 1 bis 7, **dadurch gekennzeichnet, dass** im Bereich des Displays (13) mehrere Eingabetasten (27 ... 86) vorgesehen sind, die zum Empfangen einer Benutzereingabe konfiguriert sind wobei das Display (13) schaltlogisch mit den Tasten (27 - 86) verbunden ist und mehrere zugeordnete Informationsfelder (37 ... 87) aufweist, wobei jedes Informationsfeld einer entsprechenden Eingabetaste zugeordnet ist und dazu konfiguriert ist, graphische Informationen anzuzeigen, die verschiedene Funktionen der entsprechenden Eingabetasten angeben, wobei das Informationsfeld (87) eingerichtet ist graphisch anzugeben, wie viele Tage mit Speicherdaten auf den externen Speicher (eine SD-Karte oder ein USB-Speicher) übertragen werden sollen.

9. Vorrichtung nach Anspruch 1 bis 8, **dadurch gekennzeichnet, dass** das Beatmungsgerät (1) zur Durchführung einer Dichtigkeitsprüfung einer Beatmungsmaske ausgebildet ist und dass ein entsprechendes Prüfungsergebnis im Bereich des Displays (13) visualisierbar ist.

10. Vorrichtung nach Anspruch 9, **dadurch gekennzeichnet, dass** ein während der Durchführung der Prüfung auf Maskendichtigkeit verwendeter Druck über das Display (13) vorgebbar ist.

11. Vorrichtung nach Anspruch 9 und/oder 10, **dadurch gekennzeichnet, dass** nach Betätigung der Eingabetaste (28), insbesondere die Durchführung eines Prüfvorganges für die Maskendichtigkeit während der Therapie im Expertenmenü erfolgt, wobei im Bereich des Displays (13) mehrere Eingabetasten (27 ...50, 51) vorgesehen sind, die zum Empfangen einer Benutzereingabe konfiguriert sind und wobei das Display (13) schaltlogisch mit den Tasten (27 - 51) verbunden ist und mehrere zugeordnete Informationsfelder (37 ... 61) aufweist wobei jedes Informationsfeld einer entsprechenden Eingabetaste zugeordnet ist und dazu konfiguriert ist, graphische Informationen anzuzeigen, die verschiedene Funktionen der entsprechenden Eingabetasten angeben, wobei zumindest ein Informationsfeld (60, 61) mit zugeordneter Eingabetaste (50, 51) vorgesehen ist, wobei das Informationsfeld (50, 51) eingerichtet ist graphisch anzugeben, ob ein einen Parameter oder eine Einstellung des Beatmungsgerätes betreffender, ausgewählter Wert (80, 81) mittels zumindest einer Eingabetaste (70, 71) verändert werden kann oder nicht und eine Veränderung dieses Zustandes über eine Betätigung der Eingabetaste (61) erreicht werden kann, die dem Informationsfeld (61) zugeordnet ist, wobei nach einer entsprechenden Betätigung der Eingabetaste (61) das Informationsfeld (60) einen geschlossenen Zustand visualisiert und zumindest ausgewählte Werte (80, 81), die den Betrieb des Beatmungsgerätes betreffen, daraufhin nicht mehr veränderbar sind.

12. Vorrichtung nach Anspruch 1 bis 11, **dadurch gekennzeichnet, dass** über das Display (13) mindestens eine Energiesparoption auswählbar und konfigurierbar ist.

13. Vorrichtung nach Anspruch 1 bis 12, **dadurch gekennzeichnet, dass** über das Display (13) mindestens eine Formation zur Gerätefunktion und/oder Gerätebedienung visualisierbar ist.

14. Verfahren zur Anzeige von Benutzerschnittstelleninformationen für ein Beatmungsgerät, welches ein mit einer Steuerung verbundenes Gebläse aufweist, wobei sowohl die Steuerung als auch das Gebläse in einem Gehäuse angeordnet sind, und wobei die Steuerung mit mindestens einer Anzeigeeinrichtung sowie mindestens einem Bedienelement verbunden ist, **dadurch gekennzeichnet**, das die Anzeigeeinrichtung (2) als ein Display (13) ausgebildet ist, dass eine Touchscreen-Funktion aufweist und das unter Verwendung einer symbolbasierten Bedienstruktur derart als Eingabeeinrichtung (2) verwendbar ist, dass jeweilige Symbole eine zugeordnete Funktion visualisieren und dass von der Steuerung eine hierarchisch strukturierte Bedienbarkeit generiert ist.

15. Verfahren nach Anspruch 14 **dadurch gekennzeichnet, dass** im Bereich des Displays (13) mehrere Eingabetasten (27 ...50, 51) vorgesehen sind, die zum Empfangen einer Benutzereingabe konfiguriert sind und wobei das Display (13) schaltlogisch mit den Tasten (27 - 51) verbunden ist und mehrere zugeordnete Informationsfelder (37 ... 61) aufweist wobei jedes Informationsfeld einer entsprechenden Eingabetaste zugeordnet ist und dazu konfiguriert ist, graphische Informationen anzuzeigen, die verschiedene Funktionen der entsprechenden Eingabetasten angeben, wobei zumindest ein Informationsfeld (60, 61) mit zugeordneter Eingabetaste (50, 51) vorgesehen ist, wobei das Informationsfeld (50, 51) eingerichtet ist graphisch anzugeben, ob ein einen Parameter oder eine Einstellung des Beatmungsgerätes betreffender, ausgewählter Wert (80, 81) mittels zumindest einer Eingabetaste (70, 71) verändert werden kann.
